(19) Europäisches Patentamt
European Patent Office
Office européen des brevets

(11) EP 3 388 437 B1

(12) EUROPEAN PATENT SPECIFICATION

(45) Date of publication and mention of the grant of the patent:
**21.08.2019 Bulletin 2019/34**

(21) Application number: **18166921.9**

(22) Date of filing: **12.04.2018**

(51) Int Cl.:
***C07D 491/044*** *(2006.01)* ***C07D 495/04*** *(2006.01)*
***C09K 11/06*** *(2006.01)* ***H01L 51/50*** *(2006.01)*
***H05B 33/14*** *(2006.01)* ***H01L 51/00*** *(2006.01)*

(54) **BENZOFURO- AND BENZOTHIOPHENO-CARBAZOLE DISUBSTITUTED DICYANO BIPHENYL COMPOUNDS AND THEIR USE IN ORGANIC OPTOELECTRONIC DEVICES**

BENZOFURO- AND BENZOTHIOPHENO-CARBAZOL DISUBSTITUIERTE DICYAN BIPHENYL VERBINDUNGEN UND DEREN VERWENDUNG IN ORGANISCHEN OPTOELECTRONISCHEN VORRICHTUNGEN

COMPOSES DICYANO BIPHENYL DISUBSTITUES AVEC BENZOFURO- ET BENZOTHIOPHENO-CARBAZOLES ET LEUR UTILISATION DANS LES DISPOSITIFS ORGNIQUES OPTOELECTRONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.04.2017 EP 17166392**

(43) Date of publication of application:
**17.10.2018 Bulletin 2018/42**

(73) Proprietor: **CYNORA GMBH**
**76646 Bruchsal (DE)**

(72) Inventor: **DANZ, Michael**
**76344 Eggenstein-Leopoldshafen (DE)**

(74) Representative: **Hoppe, Georg Johannes**
**Darani Anwaltskanzlei**
**Beuckestrasse 20**
**14163 Berlin (DE)**

(56) References cited:
**EP-A1- 3 113 239**
**EP-A1- 3 141 550**
**EP-A1- 3 142 162**
**US-A1- 2017 186 974**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 388 437 B1

## Description

The invention relates to organic molecules and their use in organic light-emitting diodes (OLEDs) and in other optoelectronic devices.

EP-A-3113239 discloses OLED compounds having cyano substituents.

## Description

The object of the present invention is to provide molecules which are suitable for use in optoelectronic devices.

This object is achieved by the invention which provides a new class of organic molecules.

According to the invention, the organic molecules are purely organic molecules, i.e. they do not contain any metal ions in contrast to metal complexes known for use in organic optoelectronic devices.

According to the present invention, the organic molecules exhibit emission maxima in the blue, sky-blue or green spectral range. The organic molecules exhibit in particular emission maxima between 420 nm and 520 nm, preferably between 440 nm and 495 nm, more preferably between 450 nm and 470 nm. The photoluminescence quantum yields of the organic molecules according to the invention are, in particular, 60 % or more. The molecules according to the invention exhibit in particular thermally activated delayed fluorescence (TADF). The use of the molecules according to the invention in an optoelectronic device, for example an organic light-emitting diode (OLED), leads to higher efficiencies of the device. Corresponding OLEDs have a higher stability than OLEDs with known emitter materials and comparable color.

The organic light-emitting molecules according to the invention comprise or consist of a first chemical moiety comprising or consisting of a structure of Formula I,

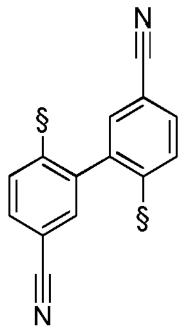

Formula I

and

- two second chemical moieties, each independently from another comprising or consisting of a structure of Formula II,

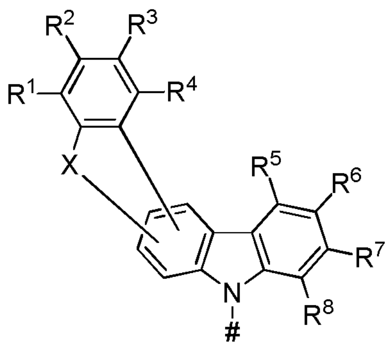

Formula II

wherein the first chemical moiety is linked to each of the two second chemical moieties via a single bond.

§ represents the binding site of a single bond linking the first chemical moiety to one of the two second chemical moieties.

# represents the binding site of a single bond linking the first chemical moiety to one of the two second chemical

moieties.

X is O or S.

$R^1$, $R^2$, $R^3$, $R^4$, $R^5$, $R^6$, $R^7$ and $R^8$ is independently from another selected from the group consisting of hydrogen,
deuterium,
$C_1$-$C_5$-alkyl,

wherein one or more hydrogen atoms are optionally substituted by deuterium; $C_6$-$C_{18}$-aryl, which is optionally substituted with one or more substituents $R^9$; and $C_3$-$C_{17}$-heteroaryl, which is optionally substituted with one or more substituents $R^9$.

$R^9$ is at each occurrence independently from another selected from the group consisting of deuterium, $C_1$-$C_5$-alkyl,

wherein one or more hydrogen atoms are optionally substituted by deuterium; and $C_6$-$C_{18}$-aryl, wherein one or more hydrogen atoms are optionally substituted by deuterium.

In one embodiment of the invention, the two second chemical moieties each at each occurrence independently from another comprise or consist of a structure of Formula IIa:

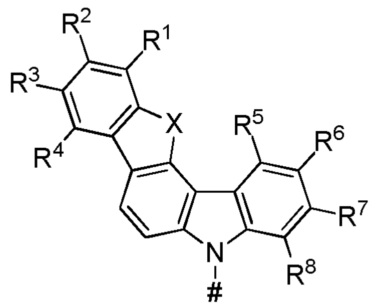

Formula IIa

wherein #, X, $R^1$, $R^2$, $R^3$, $R^4$, $R^5$, $R^6$, $R^7$ and $R^8$ are defined as above.

In one embodiment of the invention, X is O.

In one embodiment of the invention, $R^1$ selected from the group consisting of H, Me, $^i$Pr, $^t$Bu and Ph, which is optionally substituted with one or more substituents independently from each other selected from the group consisting of Me, $^i$Pr, $^t$Bu and Ph.

In one embodiment of the invention, $R^2$ selected from the group consisting of H, Me, $^i$Pr, $^t$Bu and Ph, which is optionally substituted with one or more substituents independently from each other selected from the group consisting of Me, $^i$Pr, $^t$Bu and Ph.

In one embodiment of the invention, $R^3$ selected from the group consisting of H, Me, $^i$Pr, $^t$Bu and Ph, which is optionally substituted with one or more substituents independently from each other selected from the group consisting of Me, $^i$Pr, $^t$Bu and Ph.

In one embodiment of the invention, $R^4$ selected from the group consisting of H, Me, $^i$Pr, $^t$Bu and Ph, which is optionally substituted with one or more substituents independently from each other selected from the group consisting of Me, $^i$Pr, $^t$Bu and Ph.

In one embodiment of the invention, $R^5$ selected from the group consisting of H, Me, $^i$Pr, $^t$Bu and Ph, which is optionally substituted with one or more substituents independently from each other selected from the group consisting of Me, $^i$Pr, $^t$Bu and Ph.

In one embodiment of the invention, $R^6$ selected from the group consisting of H, Me, $^i$Pr, $^t$Bu and Ph, which is optionally substituted with one or more substituents independently from each other selected from the group consisting of Me, $^i$Pr, $^t$Bu and Ph.

In one embodiment of the invention, $R^7$ selected from the group consisting of H, Me, $^i$Pr, $^t$Bu and Ph, which is optionally substituted with one or more substituents independently from each other selected from the group consisting of Me, $^i$Pr, $^t$Bu and Ph.

In one embodiment of the invention, $R^8$ selected from the group consisting of H, Me, $^i$Pr, $^t$Bu and Ph, which is optionally substituted with one or more substituents independently from each other selected from the group consisting

of Me, $^{i}$Pr, $^{t}$Bu and Ph.

In one embodiment of the invention, $R^1$, $R^2$, $R^3$, $R^4$, $R^5$, $R^6$, $R^7$ and $R^8$ is each H.

In a further embodiment of the invention, the two second chemical moieties each at each occurrence independently from another comprise or consist of a structure of Formula IIb, a structure of Formula IIb-2, a structure of Formula IIb-3 or a structure of Formula IIb-4:

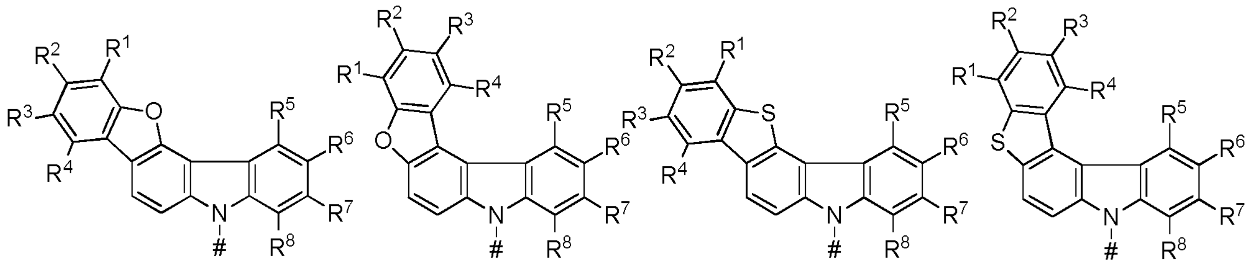

Formula IIb Formula IIb-2 Formula IIb-3 Formula IIb-4

wherein #, $R^1$, $R^2$, $R^3$, $R^4$, $R^5$, $R^6$, $R^7$ and $R^8$ are defined as above.

In a further embodiment of the invention, the two second chemical moieties each at each occurrence independently from another comprise or consist of a structure of Formula IIc, a structure of Formula IIc-2, a structure of Formula IIc-3 or a structure of Formula IIc-4, :

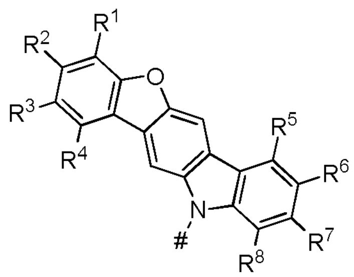

Formula IIc

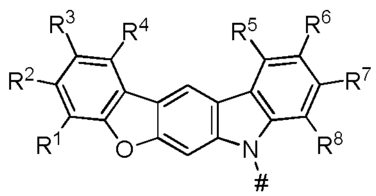

Formula IIc-2

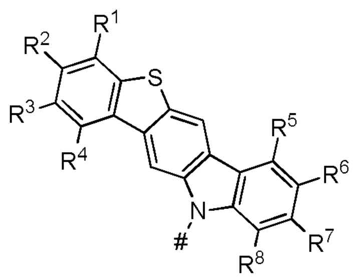

Formula IIc-3

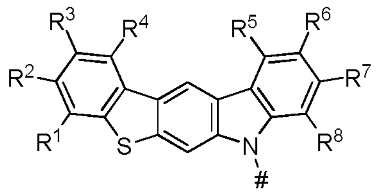

Formula IIc-4

wherein #, $R^1$, $R^2$, $R^3$, $R^4$, $R^5$, $R^6$, $R^7$ and $R^8$ are defined as above.

In a further embodiment of the invention, the two second chemical moieties each at each occurrence independently from another comprise or consist of a structure of Formula lid, a structure of Formula IId-2, a structure of Formula IId-3 or a structure of Formula IId-4, :

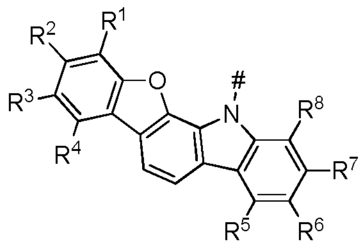

Formula IId

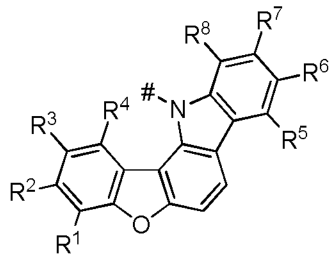

Formula IId-2

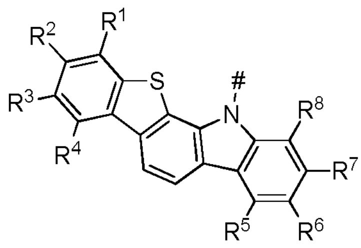

Formula IId-3

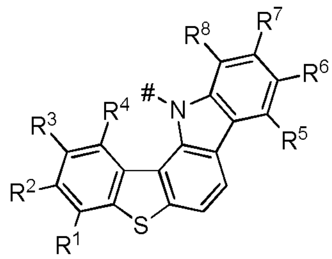

Formula IId-4

wherein #, $R^1$, $R^2$, $R^3$, $R^4$, $R^5$, $R^6$, $R^7$ and $R^8$ are defined as above.

As used throughout the present application, the terms "aryl" and "aromatic" may be understood in the broadest sense as any mono-, bi- or polycyclic aromatic moieties. Accordingly, an aryl group contains 6 to 18 aromatic ring atoms, and a heteroaryl group contains 3 to 17 aromatic ring atoms, of which at least one is a heteroatom. Notwithstanding, throughout the application the number of aromatic ring atoms may be given as subscripted number in the definition of certain substituents. In particular, the heteroaromatic ring includes one to three heteroatoms. Again, the terms "heteroaryl" and "heteroaromatic" may be understood in the broadest sense as any mono-, bi- or polycyclic hetero-aromatic moieties that include at least one heteroatom. The heteroatoms may at each occurrence be the same or different and be individually selected from the group consisting of N, O and S. Accordingly, the term "arylene" refers to a divalent substituent that bears two binding sites to other molecular structures and thereby serving as a linker structure. In case, a group in the exemplary embodiments is defined differently from the definitions given here, for example, the number of aromatic ring atoms or number of heteroatoms differs from the given definition, the definition in the exemplary embodiments is to be applied. According to the invention, a condensed (annulated) aromatic or heteroaromatic polycycle is built of two or more single aromatic or heteroaromatic cycles, which formed the polycycle via a condensation reaction.

In particular, as used throughout the present application the term aryl group or heteroaryl group comprises groups which can be bound via any position of the aromatic or heteroaromatic group, derived from benzene, naphthaline, anthracene, phenanthrene, pyrene, dihydropyrene, chrysene, fluoranthene, benzanthracene, benzphenanthrene, tetracene, furan, benzofuran, isobenzofuran, dibenzofuran, thiophene, benzothiophene, isobenzothiophene, dibenzothiophene; pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthoimidazole, phenanthroimidazole, pyridoimidazole, pyrazinoimidazole, quinoxalinoimidazole, oxazole, benzoxazole, napthooxazole, anthroxazol, phenanthroxazol, isoxazole, 1,2-thiazole, 1,3-thiazole, benzothiazole, pyridazine, benzopyridazine, pyrimidine, benzopyrimidine, 1,3,5-triazine, quinoxaline, pyrazine, phenazine, naphthyridine, carboline, benzocarboline, phenanthroline, 1,2,3-triazole, 1,2,4-triazole, benzotriazole, 1,2,3-oxadiazole, 1,2,4-oxadiazole, 1,2,5-oxadiazole, 1,2,3,4-tetrazine, purine, pteridine, indolizine und benzothiadiazole or combinations of the abovementioned groups.

As used throughout the present application the term cyclic group may be understood in the broadest sense as any mono-, bi- or polycyclic moieties.

As used throughout the present application the term alkyl group may be understood in the broadest sense as any linear, branched, or cyclic alkyl substituent. In particular, the term alkyl comprises the substituents methyl (Me), ethyl (Et), n-propyl ($^nPr$), i-propyl ($^iPr$), cyclopropyl, n-butyl ($^nBu$), i-butyl ($^iBu$), s-butyl ($^sBu$), t-butyl($^tBu$), cyclobutyl, 2-methylbutyl, n-pentyl, s-pentyl, t-pentyl, 2-pentyl, neo-pentyl, cyclopentyl, 1-methylcyclopentyl, and 2-methylpentyl.

Whenever hydrogen is mentioned herein, it could also be replaced by deuterium at each occurrence.

It is understood that when a molecular fragment is described as being a substituent or otherwise attached to

another moiety, its name may be written as if it were a fragment (e.g. naphtyl, dibenzofuryl) or as if it were the whole molecule (e.g. naphthalene, dibenzofuran). As used herein, these different ways of designating a substituent or attached fragment are considered to be equivalent.

In one embodiment, the organic molecules according to the invention have an excited state lifetime of not more than 100 μs, of not more than 70 μs, in particular of not more than 50 μs, more preferably of not more than 10 μs or not more than 7 μs in a film of poly(methyl methacrylate) (PMMA) with 10 % by weight of organic molecule at room temperature.

In one embodiment, the organic molecules according to the invention have an excited state lifetime of not more than 100 μs, of not more than 70 μs, in particular of not more than 50 μs, more preferably of not more than 10 μs or not more than 7 μs in a film of Bis[2-(diphenylphosphino)phenyl] ether oxide (DPEPO) with 10% by weight of organic molecule at room temperature.

In one embodiment of the invention, the organic molecules according to the invention represent thermally-activated delayed fluorescence (TADF) emitters, which exhibit a $\Delta E_{ST}$ value, which corresponds to the energy difference between the first excited singlet state (S1) and the first excited triplet state (T1), of less than 5000 $cm^{-1}$, preferably less than 3000 $cm^{-1}$, more preferably less than 1500 $cm^{-1}$, even more preferably less than 1000 $cm^{-1}$ or even less than 500 $cm^{-1}$.

In a further embodiment of the invention, the organic molecules according to the invention have an emission peak in the visible or nearest ultraviolet range, i.e., in the range of a wavelength of from 380 to 800 nm, with a full width at half maximum of less than 0.45 eV, preferably less than 0.42 eV, more preferably less than 0.38 eV, even more preferably less than 0.36 eV or even less than 0.34 eV in a film of poly(methyl methacrylate) (PMMA) with 10% by weight of organic molecule at room temperature.

In a further embodiment of the invention, the organic molecules according to the invention have a "blue material index" (BMI), calculated by dividing the photoluminescence quantum yield (PLQY) in % by the CIEy color coordinate of the emitted light, of more than 150, in particular more than 200, preferably more than 300, more preferably of more than 500 or even more than 700.

Orbital and excited state energies can be determined either by means of experimental methods or by calculations employing quantum-chemical methods, in particular density functional theory calculations. The energy of the highest occupied molecular orbital $E^{HOMO}$ is determined by methods known to the person skilled in the art from cyclic voltammetry measurements with an accuracy of 0.1 eV. The energy of the lowest unoccupied molecular orbital $E^{LUMO}$ is calculated as $E^{HOMO} + E^{gap}$, wherein $E^{gap}$ is determined as follows: For host compounds, the onset of the emission spectrum of a film with 10% by weight of host in poly(methyl methacrylate) (PMMA) is used as $E^{gap}$, unless stated otherwise. For emitter molecules, $E^{gap}$ is determined as the energy at which the excitation and emission spectra of a film with 10% by weight of emitter in PMMA cross.

The energy of the first excited triplet state T1 is determined from the onset of the emission spectrum at low temperature, typically at 77 K. For host compounds, where the first excited singlet state and the lowest triplet state are energetically separated by > 0.4 eV, the phosphorescence is usually visible in a steady-state spectrum in 2-Me-THF. The triplet energy can thus be determined as the onset of the phosphorescence spectrum. For TADF emitter molecules, the energy of the first excited triplet state T1 is determined from the onset of the delayed emission spectrum at 77 K, if not otherwise stated measured in a film of) PMMA with 10% by weight of emitter. Both for host and emitter compounds, the energy of the first excited singlet state S1 is determined from the onset of the emission spectrum, if not otherwise stated measured in a film of PMMA with 10% by weight of host or emitter compound.

The onset of an emission spectrum is determined by computing the intersection of the tangent to the emission spectrum with the x-axis. The tangent to the emission spectrum is set at the high-energy side of the emission band and at the point at half maximum of the maximum intensity of the emission spectrum.

A further aspect of the invention relates to the use of an organic molecule according to the invention as a luminescent emitter or as an absorber, and/or as host material and/or as electron transport material, and/or as hole injection material, and/or as hole blocking material in an organic optoelectronic device.

The organic optoelectronic device may be understood in the broadest sense as any device based on organic materials that is suitable for emitting light in the visible or nearest ultraviolet (UV) range, i.e., in the range of a wavelength of from 380 to 800 nm. More preferably, organic optoelectronic device may be able to emit light in the visible range, i.e., of from 400 to 800 nm.

In the context of such use, the organic optoelectronic device is more particularly selected from the group consisting of:

- organic light-emitting diodes (OLEDs),
- light-emitting electrochemical cells,
- OLED sensors, especially in gas and vapor sensors not hermetically externally shielded,
- organic diodes,
- organic solar cells,

- organic transistors,
- organic field-effect transistors,
- organic lasers and
- down-conversion elements.

In a preferred embodiment in the context of such use, the organic optoelectronic device is a device selected from the group consisting of an organic light emitting diode (OLED), a light emitting electrochemical cell (LEC), and a light-emitting transistor.
In the case of the use, the fraction of the organic molecule according to the invention in the emission layer in an organic optoelectronic device, more particularly in OLEDs, is 1 % to 99 % by weight, more particularly 5 % to 80 % by weight. In an alternative embodiment, the proportion of the organic molecule in the emission layer is 100 % by weight.
In one embodiment, the light-emitting layer comprises not only the organic molecules according to the invention but also a host material whose triplet (T1) and singlet (S1) energy levels are energetically higher than the triplet (T1) and singlet (S1) energy levels of the organic molecule.
A further aspect of the invention relates to a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

In one embodiment, the light-emitting layer comprises (or (essentially) consists of) a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

Particularly preferably the light-emitting layer EML comprises (or (essentially) consists of) a composition comprising or consisting of:

(i) 1-50 % by weight, preferably 5-40 % by weight, in particular 10-30 % by weight, of one or more organic molecules according to the invention;
(ii) 5-99 % by weight, preferably 30-94.9 % by weight, in particular 40-89% by weight, of at least one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

Preferably, energy can be transferred from the host compound H to the one or more organic molecules according to the invention, in particular transferred from the first excited triplet state T1(H) of the host compound H to the first excited triplet state T1(E) of the one or more organic molecules according to the invention and/ or from the first excited singlet state S1(H) of the host compound H to the first excited singlet state S1(E) of the one or more organic molecules according to the invention.
In a further embodiment, the light-emitting layer EML comprises (or (essentially) consists of) a composition comprising or consisting of:

(i) 1-50 % by weight, preferably 5-40 % by weight, in particular 10-30 % by weight, of one organic molecule according to the invention;
(ii) 5-99 % by weight, preferably 30-94.9 % by weight, in particular 40-89% by weight, of one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

In one embodiment, the host compound H has a highest occupied molecular orbital HOMO(H) having an energy

$E^{HOMO}(H)$ in the range of from -5 to -6.5 eV and the at least one further host compound D has a highest occupied molecular orbital HOMO(D) having an energy $E^{HOMO}(D)$, wherein $E^{HOMO}(H) > E^{HOMO}(D)$.

In a further embodiment, the host compound H has a lowest unoccupied molecular orbital LUMO(H) having an energy $E^{LUMO}(H)$ and the at least one further host compound D has a lowest unoccupied molecular orbital LUMO(D) having an energy $E^{LUMO}(D)$, wherein $E^{LUMO}(H) > E^{LUMO}(D)$.

In one embodiment, the host compound H has a highest occupied molecular orbital HOMO(H) having an energy $E^{HOMO}(H)$ and a lowest unoccupied molecular orbital LUMO(H) having an energy $E^{LUMO}(H)$, and

the at least one further host compound D has a highest occupied molecular orbital HOMO(D) having an energy $E^{HOMO}(D)$ and a lowest unoccupied molecular orbital LUMO(D) having an energy $E^{LUMO}(D)$,
the organic molecule according to the invention has a highest occupied molecular orbital HOMO(E) having an energy $E^{HOMO}(E)$ and a lowest unoccupied molecular orbital LUMO(E) having an energy $E^{LUMO}(E)$,
wherein

$E^{HOMO}(H) > E^{HOMO}(D)$ and the difference between the energy level of the highest occupied molecular orbital HOMO(E) of organic molecule according to the invention ($E^{HOMO}(E)$) and the energy level of the highest occupied molecular orbital HOMO(H) of the host compound H ($E^{HOMO}(H)$) is between -0.5 eV and 0.5 eV, more preferably between -0.3 eV and 0.3 eV, even more preferably between -0.2 eV and 0.2 eV or even between -0.1 eV and 0.1 eV; and
$E^{LUMO}(H) > E^{LUMO}(D)$ and the difference between the energy level of the lowest unoccupied molecular orbital LUMO(E) of organic molecule according to the invention ($E^{LUMO}(E)$) and the lowest unoccupied molecular orbital LUMO(D) of the at least one further host compound D ($E^{LUMO}(D)$) is between -0.5 eV and 0.5 eV, more preferably between -0.3 eV and 0.3 eV, even more preferably between -0.2 eV and 0.2 eV or even between -0.1 eV and 0.1 eV.

In a further aspect, the invention relates to an organic optoelectronic device comprising an organic molecule or a composition of the type described here, more particularly in the form of a device selected from the group consisting of organic light-emitting diode (OLED), light-emitting electrochemical cell, OLED sensor, more particularly gas and vapor sensors not hermetically externally shielded, organic diode, organic solar cell, organic transistor, organic field-effect transistor, organic laser and down-conversion element.

In a preferred embodiment, the organic optoelectronic device is a device selected from the group consisting of an organic light emitting diode (OLED), a light emitting electrochemical cell (LEC), and a light-emitting transistor.

In one embodiment of the organic optoelectronic device of the invention, the organic molecule according to the invention is used as emission material in a light-emitting layer EML.

In one embodiment of the organic optoelectronic device of the invention the light-emitting layer EML consists of the composition according to the invention described here.

Exemplarily, when the organic optoelectronic device is an OLED, it may exhibit the following layer structure:

1. substrate
2. anode layer A
3. hole injection layer, HIL
4. hole transport layer, HTL
5. electron blocking layer, EBL
6. emitting layer, EML
7. hole blocking layer, HBL
8. electron transport layer, ETL
9. electron injection layer, EIL
10. cathode layer,

wherein the OLED comprises each layer only optionally, different layers may be merged and the OLED may comprise more than one layer of each layer type defined above.

Furthermore, the organic optoelectronic device may optionally comprise one or more protective layers protecting the device from damaging exposure to harmful species in the environment including, exemplarily moisture, vapor and/or gases.

In one embodiment of the invention, the organic optoelectronic device is an OLED, which exhibits the following inverted layer structure:

1. substrate

2. cathode layer
3. electron injection layer, EIL
4. electron transport layer, ETL
5. hole blocking layer, HBL
6. emitting layer, B
7. electron blocking layer, EBL
8. hole transport layer, HTL
9. hole injection layer, HIL
10. anode layer A

Wherein the OLED with an inverted layer structure comprises each layer only optionally, different layers may be merged and the OLED may comprise more than one layer of each layer types defined above.

In one embodiment of the invention, the organic optoelectronic device is an OLED, which may exhibit stacked architecture. In this architecture, contrary to the typical arrangement, where the OLEDs are placed side by side, the individual units are stacked on top of each other. Blended light may be generated with OLEDs exhibiting a stacked architecture, in particular white light may be generated by stacking blue, green and red OLEDs. Furthermore, the OLED exhibiting a stacked architecture may optionally comprise a charge generation layer (CGL), which is typically located between two OLED subunits and typically consists of a n-doped and p-doped layer with the n-doped layer of one CGL being typically located closer to the anode layer.

In one embodiment of the invention, the organic optoelectronic device is an OLED, which comprises two or more emission layers between anode and cathode. In particular, this so-called tandem OLED comprises three emission layers, wherein one emission layer emits red light, one emission layer emits green light and one emission layer emits blue light, and optionally may comprise further layers such as charge generation layers, blocking or transporting layers between the individual emission layers. In a further embodiment, the emission layers are adjacently stacked. In a further embodiment, the tandem OLED comprises a charge generation layer between each two emission layers. In addition, adjacent emission layers or emission layers separated by a charge generation layer may be merged.

The substrate may be formed by any material or composition of materials. Most frequently, glass slides are used as substrates. Alternatively, thin metal layers (e.g., copper, gold, silver or aluminum films) or plastic films or slides may be used. This may allow a higher degree of flexibility. The anode layer A is mostly composed of materials allowing to obtain an (essentially) transparent film. As at least one of both electrodes should be (essentially) transparent in order to allow light emission from the OLED, either the anode layer A or the cathode layer C is transparent. Preferably, the anode layer A comprises a large content or even consists of transparent conductive oxides (TCOs). Such anode layer A may exemplarily comprise indium tin oxide, aluminum zinc oxide, fluorine doped tin oxide, indium zinc oxide, PbO, SnO, zirconium oxide, molybdenum oxide, vanadium oxide, wolfram oxide, graphite, doped Si, doped Ge, doped GaAs, doped polyaniline, doped polypyrrol and/or doped polythiophene.

Particularly preferably, the anode layer A (essentially) consists of indium tin oxide (ITO) (e.g., (InO3)0.9(SnO2)0.1). The roughness of the anode layer A caused by the transparent conductive oxides (TCOs) may be compensated by using a hole injection layer (HIL). Further, the HIL may facilitate the injection of quasi charge carriers (i.e., holes) in that the transport of the quasi charge carriers from the TCO to the hole transport layer (HTL) is facilitated. The hole injection layer (HIL) may comprise poly-3,4-ethylendioxy thiophene (PEDOT), polystyrene sulfonate (PSS), $MoO_2$, $V_2O_5$, CuPC or CuI, in particular a mixture of PEDOT and PSS. The hole injection layer (HIL) may also prevent the diffusion of metals from the anode layer A into the hole transport layer (HTL). The HIL may exemplarily comprise PEDOT:PSS (poly-3,4-ethylendioxy thiophene: polystyrene sulfonate), PEDOT (poly-3,4-ethylendioxy thiophene), mMTDATA (4,4',4"-tris[phenyl(m-tolyl)amino]triphenylamine), Spiro-TAD (2,2',7,7'-tetrakis(n,n-diphenylamino)-9,9'-spirobifluorene), DNTPD (N1,N1'-(biphenyl-4,4'-diyl)bis(N1-phenyl-N4,N4-di-m-tolylbenzene-1,4-diamine), NPB (N,N'-nis-(1-naphthalenyl)-N,N'-bis-phenyl-(1,1'-biphenyl)-4,4'-diamine), NPNPB (N,N'-diphenyl-N,N'-di-[4-(N,N-diphenyl-amino)phenyl]benzidine), MeO-TPD (N,N,N',N'-tetrakis(4-methoxyphenyl)benzidine), HAT-CN (1,4,5,8,9,11-hexaazatriphenylen-hexacarbonitrile) and/or Spiro-NPD (N,N'-diphenyl-N,N'-bis-(1-naphthyl)-9,9'-spirobifluorene-2,7-diamine).

Adjacent to the anode layer A or hole injection layer (HIL) typically a hole transport layer (HTL) is located. Herein, any hole transport compound may be used. Exemplarily, electron-rich heteroaromatic compounds such as triarylamines and/or carbazoles may be used as hole transport compound. The HTL may decrease the energy barrier between the anode layer A and the light-emitting layer EML. The hole transport layer (HTL) may also be an electron blocking layer (EBL). Preferably, hole transport compounds bear comparably high energy levels of their triplet states T1. Exemplarily the hole transport layer (HTL) may comprise a star-shaped heterocycle such as tris(4-carbazoyl-9-ylphenyl)amine (TCTA), poly-TPD (poly(4-butylphenyl-diphenyl-amine)), [alpha]-NPD (poly(4-butylphenyl-diphenyl-amine)), TAPC (4,4'-cyclohexyliden-bis[N,N-bis(4-methylphenyl)benzenamine]), 2-TNATA (4,4',4"-tris[2-naphthyl(phenyl)amino]triphenylamine), Spiro-TAD, DNTPD, NPB, NPNPB, MeO-TPD, HAT-CN and/or TrisPcz (9,9'-diphenyl-6-(9-phenyl-9H-carbazol-3-yl)-9H,9'H-3,3'-bicarbazole). In addition, the HTL may comprise a p-doped layer, which may be

composed of an inorganic or organic dopant in an organic hole-transporting matrix. Transition metal oxides such as vanadium oxide, molybdenum oxide or tungsten oxide may exemplarily be used as inorganic dopant. Tetrafluorotetracyanoquinodimethane (F4-TCNQ), copper-pentafluorobenzoate (Cu(I)pFBz) or transition metal complexes may exemplarily be used as organic dopant.

The EBL may exemplarily comprise mCP (1,3-bis(carbazol-9-yl)benzene), TCTA, 2-TNATA, mCBP (3,3-di(9H-carbazol-9-yl)biphenyl), tris-Pcz, CzSi (9-(4-tert-Butylphenyl)-3,6-bis(triphenylsilyl)-9H-carbazole), and/or DCB (N,N'-dicarbazolyl-1,4-dimethylbenzene).

Adjacent to the hole transport layer (HTL), typically, the light-emitting layer EML is located. The light-emitting layer EML comprises at least one light emitting molecule. Particular, the EML comprises at least one light emitting molecule according to the invention. In one embodiment, the light-emitting layer comprises only the organic molecules according to the invention. Typically, the EML additionally comprises one or more host material. Exemplarily, the host material is selected from CBP (4,4'-Bis-(N-carbazolyl)-biphenyl), mCP, mCBP Sif87 (dibenzo[b,d]thiophen-2-yltriphenylsilane), CzSi, Sif88 (dibenzo[b,d]thiophen-2-yl)diphenylsilane), DPEPO (bis[2-(diphenylphosphino)phenyl] ether oxide), 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole, 9-[3,5-bis(2-dibenzothiophenyl)phenyl]-9H-carbazole, T2T (2,4,6-tris(biphenyl-3-yl)-1,3,5-triazine), T3T (2,4,6-tris(triphenyl-3-yl)-1,3,5-triazine) and/or TST (2,4,6-tris(9,9'-spirobifluorene-2-yl)-1,3,5-triazine). The host material typically should be selected to exhibit first triplet (T1) and first singlet (S1) energy levels, which are energetically higher than the first triplet (T1) and first singlet (S1) energy levels of the organic molecule.

In one embodiment of the invention, the EML comprises a so-called mixed-host system with at least one hole-dominant host and one electron-dominant host. In a particular embodiment, the EML comprises exactly one light emitting molecule species according to the invention and a mixed-host system comprising T2T as electron-dominant host and a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole as hole-dominant host. In a further embodiment the EML comprises 50-80 % by weight, preferably 60-75 % by weight of a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole; 10-45 % by weight, preferably 15-30 % by weight of T2T and 5-40 % by weight, preferably 10-30 % by weight of light emitting molecule according to the invention.

Adjacent to the light-emitting layer EML an electron transport layer (ETL) may be located. Herein, any electron transporter may be used. Exemplarily, compounds poor of electrons such as, e.g., benzimidazoles, pyridines, triazoles, oxadiazoles (e.g., 1,3,4-oxadiazole), phosphinoxides and sulfone, may be used. An electron transporter may also be a star-shaped heterocycle such as 1,3,5-tri(1-phenyl-1H-benzo[d]imidazol-2-yl)phenyl (TPBi). The ETL may comprise NBphen (2,9-bis(naphthalen-2-yl)-4,7-diphenyl-1,10-phenanthroline), Alq3 (Aluminum-tris(8-hydroxyquinoline)), TSPO1 (diphenyl-4-triphenylsilylphenyl-phosphinoxide), BPyTP2 (2,7-di(2,2'-bipyridin-5-yl)triphenyle), Sif87 (dibenzo[b,d]thiophen-2-yltriphenylsilane), Sif88 (dibenzo[b,d]thiophen-2-yl)diphenylsilane), BmPyPhB (1,3-bis[3,5-di(pyridin-3-yl)phenyl]benzene) and/or BTB (4,4'-bis-[2-(4,6-diphenyl-1,3,5-triazinyl)]-1,1'-biphenyl). Optionally, the ETL may be doped with materials such as Liq. The electron transport layer (ETL) may also block holes or a holeblocking layer (HBL) is introduced.

The HBL may exemplarily comprise BCP (2,9-dimethyl-4,7-diphenyl-1,10-phenanthroline = Bathocuproine), BAlq (bis(8-hydroxy-2-methylquinoline)-(4-phenylphenoxy)aluminum), NBphen (2,9-bis(naphthalen-2-yl)-4,7-diphenyl-1,10-phenanthroline), Alq3 (Aluminum-tris(8-hydroxyquinoline)), TSPO1 (diphenyl-4-triphenylsilylphenyl-phosphinoxide), T2T (2,4,6-tris(biphenyl-3-yl)-1,3,5-triazine), T3T (2,4,6-tris(triphenyl-3-yl)-1,3,5-triazine), TST (2,4,6-tris(9,9'-spirobifluorene-2-yl)-1,3,5-triazine), and/or TCB/TCP (1,3,5-tris(N-carbazolyl)benzol/ 1,3,5-tris(carbazol)-9-yl) benzene).

Adjacent to the electron transport layer (ETL), a cathode layer C may be located. Exemplarily, the cathode layer C may comprise or may consist of a metal (e.g., Al, Au, Ag, Pt, Cu, Zn, Ni, Fe, Pb, LiF, Ca, Ba, Mg, In, W, or Pd) or a metal alloy. For practical reasons, the cathode layer may also consist of (essentially) non-transparent metals such as Mg, Ca or Al. Alternatively or additionally, the cathode layer C may also comprise graphite and or carbon nanotubes (CNTs). Alternatively, the cathode layer C may also consist of nanoscalic silver wires.

An OLED may further, optionally, comprise a protection layer between the electron transport layer (ETL) and the cathode layer C (which may be designated as electron injection layer (EIL)). This layer may comprise lithium fluoride, cesium fluoride, silver, Liq (8-hydroxyquinolinolatolithium), $Li_2O$, $BaF_2$, MgO and/or NaF.

Optionally, also the electron transport layer (ETL) and/or a hole blocking layer (HBL) may comprise one or more host compounds.

In order to modify the emission spectrum and/or the absorption spectrum of the light-emitting layer EML further, the light-emitting layer EML may further comprise one or more further emitter molecule F. Such an emitter molecule F may be any emitter molecule known in the art. Preferably such an emitter molecule F is a molecule with a structure

differing from the structure of the molecules according to the invention. The emitter molecule F may optionally be a TADF emitter. Alternatively, the emitter molecule F may optionally be a fluorescent and/or phosphorescent emitter molecule which is able to shift the emission spectrum and/or the absorption spectrum of the light-emitting layer EML. Exemplarily, the triplet and/or singlet excitons may be transferred from the emitter molecule according to the invention to the emitter molecule F before relaxing to the ground state S0 by emitting light typically red-shifted in comparison to the light emitted by emitter molecule E. Optionally, the emitter molecule F may also provoke two-photon effects (i.e., the absorption of two photons of half the energy of the absorption maximum).

Optionally, an organic optoelectronic device (e.g., an OLED) may exemplarily be an essentially white organic optoelectronic device. Exemplarily such white organic optoelectronic device may comprise at least one (deep) blue emitter molecule and one or more emitter molecules emitting green and/or red light. Then, there may also optionally be energy transmittance between two or more molecules as described above.

As used herein, if not defined more specifically in the particular context, the designation of the colors of emitted and/or absorbed light is as follows:

| | |
|---|---|
| violet: | wavelength range of >380-420 nm; |
| deep blue: | wavelength range of >420-480 nm; |
| sky blue: | wavelength range of >480-500 nm; |
| green: | wavelength range of >500-560 nm; |
| yellow: | wavelength range of >560-580 nm; |
| orange: | wavelength range of >580-620 nm; |
| red: | wavelength range of >620-800 nm |

With respect to emitter molecules, such colors refer to the emission maximum. Therefore, exemplarily, a deep blue emitter has an emission maximum in the range of from >420 to 480 nm, a sky blue emitter has an emission maximum in the range of from >480 to 500 nm, a green emitter has an emission maximum in a range of from >500 to 560 nm, a red emitter has an emission maximum in a range of from >620 to 800 nm.

A deep blue emitter may preferably have an emission maximum of below 480 nm, more preferably below 470 nm, even more preferably below 465 nm or even below 460 nm. It will typically be above 420 nm, preferably above 430 nm, more preferably above 440 nm or even above 445 nm.

Accordingly, a further aspect of the present invention relates to an OLED, which exhibits an external quantum efficiency at 1000 cd/$m^2$ of more than 8%, more preferably of more than 10%, more preferably of more than 13%, even more preferably of more than 15% or even more than 20% and/or exhibits an emission maximum between 420 nm and 500 nm, preferably between 430 nm and 490 nm, more preferably between 440 nm and 480 nm, even more preferably between 445 nm and 460 nm and/or exhibits a LT80 value at 500 cd/$m^2$ of more than 100 h, preferably more than 200 h, more preferably more than 400 h, even more preferably more than 750 h or even more than 1000 h. Accordingly, a further aspect of the present invention relates to an OLED, whose emission exhibits a CIEy color coordinate of less than 0.45, preferably less than 0.30, more preferably less than 0.20 or even more preferably less than 0.15 or even less than 0.10.

A further aspect of the present invention relates to an OLED, which emits light at a distinct color point. According to the present invention, the OLED emits light with a narrow emission band (small full width at half maximum (FWHM)). In one aspect, the OLED according to the invention emits light with a FWHM of the main emission peak of less than 0.45 eV, preferably less than 0.42 eV, more preferably less than 0.38 eV, even more preferably less than 0.36 eV or even less than 0.34 eV.

A further aspect of the present invention relates to an OLED, which emits light with CIEx and CIEy color coordinates close to the CIEx (= 0.131) and CIEy (= 0.046) color coordinates of the primary color blue (CIEx = 0.131 and CIEy = 0.046) as defined by ITU-R Recommendation BT.2020 (Rec. 2020) and thus is suited for the use in Ultra High Definition (UHD) displays, e.g. UHD-TVs. Accordingly, a further aspect of the present invention relates to an OLED, whose emission exhibits a CIEx color coordinate of between 0.02 and 0.30, preferably between 0.03 and 0.25, more preferably between 0.05 and 0.20 or even more preferably between 0.08 and 0.18 or even between 0.10 and 0.15 and/or a CIEy color coordinate of between 0.00 and 0.45, preferably between 0.01 and 0.30, more preferably between 0.02 and 0.20 or even more preferably between 0.03 and 0.15 or even between 0.04 and 0.10.

In a further aspect, the invention relates to a method for producing an optoelectronic component. In this case an organic molecule of the invention is used.

The organic optoelectronic device, in particular the OLED according to the present invention can be produced by any means of vapor deposition and/ or liquid processing. Accordingly, at least one layer is

- prepared by means of a sublimation process,
- prepared by means of an organic vapor phase deposition process,
- prepared by means of a carrier gas sublimation process,
- solution processed or printed.

The methods used to produce the organic optoelectronic device, in particular the OLED according to the present invention are known in the art. The different layers are individually and successively deposited on a suitable substrate by means of subsequent deposition processes. The individual layers may be deposited using the same or differing deposition methods.

Vapor deposition processes exemplarily comprise thermal (co)evaporation, chemical vapor deposition and physical vapor deposition. For active matrix OLED display, an AMOLED backplane is used as substrate. The individual layer may be processed from solutions or dispersions employing adequate solvents. Solution deposition process exemplarily comprise spin coating, dip coating and jet printing. Liquid processing may optionally be carried out in an inert atmosphere (e.g., in a nitrogen atmosphere) and the solvent may optionally be completely or partially removed by means known in the state of the art.

Examples

General Synthesis Scheme I

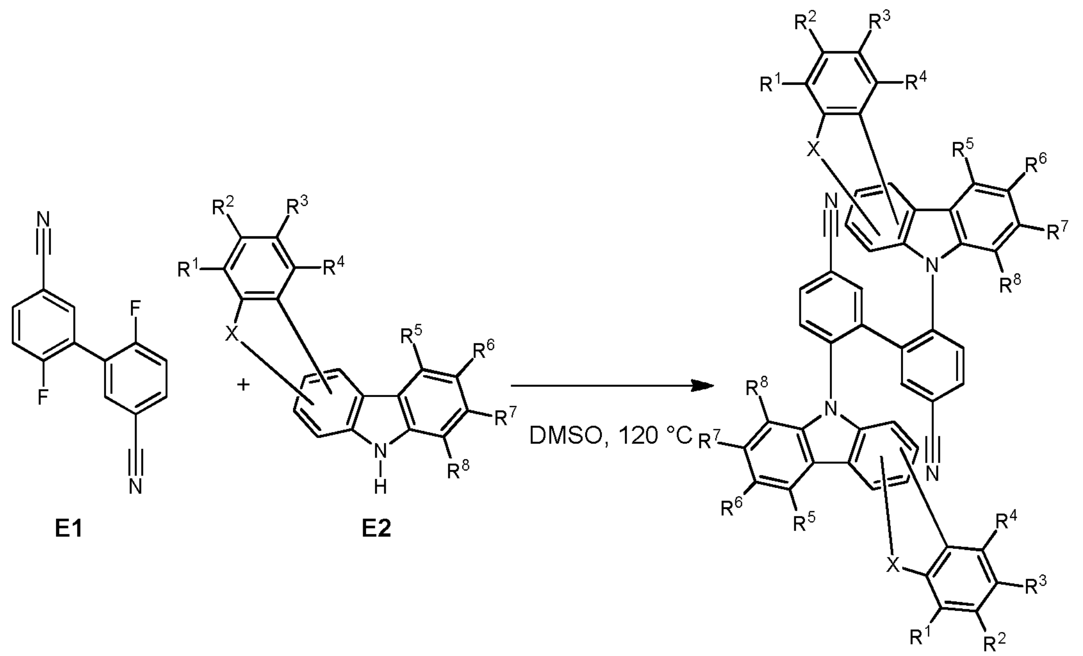


**E1** **E2**

*General procedure for synthesis:*

**E1** (1 equivalent each), **E2** (2.10 equivalents) and tribasic potassium phosphate (4.00 equivalents) are suspended under nitrogen atmosphere in DMSO and stirred at 120 °C (16 h). Subsequently the reaction mixture is poured into an ice/water mixture. The resulting solid is filtrated by suction. After drying the solid in vacuum, the crude product is purified by recrystallization out of toluene or by flash chromatography. The product is obtained as a solid.

**HPLC-MS:**

HPLC-MS spectroscopy is performed on a HPLC by Agilent (1100 series) with MS-detector (Thermo LTQ XL). A reverse phase column 4,6mm x 150mm, particle size 5,0μm from Waters (without pre-column) is used in the HPLC. The HPLC-MS measurements are performed at room temperature (rt) with the solvents acetonitrile, water and THF in the following concentrations:

| | |
|---|---|
| solvent A: | $H_2O$ (90%) MeCN (10%) |
| solvent B: | $H_2O$ (10%) MeCN (90%) |
| solvent C: | THF (100%) |

From a solution with a concentration of 0.5mg/ml an injection volume of 15 μL is taken for the measurements. The following gradient is used:

| Flow rate [ml/min] | time [min] | A[%] | B[%] | D[%] |
|---|---|---|---|---|
| 3 | 0 | 40 | 50 | 10 |
| 3 | 10 | 10 | 15 | 75 |
| 3 | 16 | 10 | 15 | 75 |
| 3 | 16.01 | 40 | 50 | 10 |
| 3 | 20 | 40 | 50 | 10 |

Ionisation of the probe is performed by APCI (atmospheric pressure chemical ionization).

*Cyclic voltammetry*

Cyclic voltammograms are measured from solutions having concentration of $10^{-3}$ mol/l of the organic molecules in dichloromethane or a suitable solvent and a suitable supporting electrolyte (e.g. 0.1 mol/l of tetrabutylammonium hexafluorophosphate). The measurements are conducted at room temperature under nitrogen atmosphere with a three-electrode assembly (Working and counter electrodes: Pt wire, reference electrode: Pt wire) and calibrated using $FeCp_2/FeCp_2^+$ as internal standard. The HOMO data was corrected using ferrocene as internal standard against a saturated calomel electrode (SCE).

*Density functional theory calculation*

Molecular structures are optimized employing the BP86 functional and the resolution of identity approach (RI). Excitation energies are calculated using the (BP86) optimized structures employing Time-Dependent DFT (TD-DFT) methods. Orbital and excited state energies are calculated with the B3LYP functional. Def2-SVP basis sets (and a m4-grid for numerical integration are used. The Turbomole program package is used for all calculations.

*Photophysical measurements*

Sample pretreatment: Spin-coating
Apparatus: Spin150, SPS euro.
The sample concentration is 10 mg/ml, dissolved in a suitable solvent.
Program: 1) 3 s at 400 U/min; 20 s at 1000 U/min at 1000 Upm/s. 3) 10 s at 4000 U/min at 1000 Upm/s. After coating, the films are tried at 70 °C for 1 min.

Photoluminescence spectroscopy and TCSPC (*Time-correlated single-photon counting*) Steady-state emission spectroscopy is measured by a Horiba Scientific, Modell FluoroMax-4 equipped with a 150 W Xenon-Arc lamp, excitation- and emissions monochromators and a Hamamatsu R928 photomultiplier and a time-correlated single-photon counting option. Emissions and excitation spectra are corrected using standard correction fits.

Excited state lifetimes are determined employing the same system using the TCSPC method with FM-2013 equipment and a Horiba Yvon TCSPC hub.

Excitation sources:

NanoLED 370 (wavelength: 371 nm, puls duration: 1,1 ns)
NanoLED 290 (wavelength: 294 nm, puls duration: <1 ns)

SpectraLED 310 (wavelength: 314 nm)
SpectraLED 355 (wavelength: 355 nm).

Data analysis (exponential fit) is done using the software suite DataStation and DAS6 analysis software. The fit is specified using the chi-squared-test.

Photoluminescence quantum yield measurements

For photoluminescence quantum yield (PLQY) measurements an *Absolute PL Quantum Yield Measurement C9920-03G* system (*Hamamatsu Photonics*) is used. Quantum yields and CIE coordinates are determined using the software U6039-05 version 3.6.0.

Emission maxima are given in nm, quantum yields Φ in % and CIE coordinates as x,y values. PLQY is determined using the following protocol:

1) Quality assurance: Anthracene in ethanol (known concentration) is used as reference
2) Excitation wavelength: the absorption maximum of the organic molecule is determined and the molecule is excited using this wavelength
3) Measurement
Quantum yields are measured for sample of solutions or films under nitrogen atmosphere. The yield is calculated using the equation:

$$\Phi_{PL} = \frac{n_{photon}, emited}{n_{photon}, absorbed} = \frac{\int \frac{\lambda}{hc}\left[Int_{emitted}^{sample}(\lambda) - Int_{absorbed}^{sample}(\lambda)\right]d\lambda}{\int \frac{\lambda}{hc}\left[Int_{emitted}^{reference}(\lambda) - Int_{absorbed}^{reference}(\lambda)\right]d\lambda}$$

wherein $n_{photon}$ denotes the photon count and Int. the intensity.

**Production and characterization of organic optoelectronic devices**

OLED devices comprising organic molecules according to the invention can be produced via vacuum-deposition methods. If a layer contains more than one compound, the weight-percentage of one or more compounds is given in %. The total weight-percentage values amount to 100 %, thus if a value is not given, the fraction of this compound equals to the difference between the given values and 100%.

The not fully optimized OLEDs are characterized using standard methods and measuring electroluminescence spectra, the external quantum efficiency (in %) in dependency on the intensity, calculated using the light detected by the photodiode, and the current. The OLED device lifetime is extracted from the change of the luminance during operation at constant current density. The LT50 value corresponds to the time, where the measured luminance decreased to 50 % of the initial luminance, analogously LT80 corresponds to the time point, at which the measured luminance decreased to 80 % of the initial luminance, LT 95 to the time point, at which the measured luminance decreased to 95 % of the initial luminance etc.

Accelerated lifetime measurements are performed (e.g. applying increased current densities).

Exemplarily LT80 values at 500 cd/m$^2$ are determined using the following equation:

$$\mathrm{LT80}\left(500\frac{cd^2}{m^2}\right) = \mathrm{LT80}(L_0)\left(\frac{L_0}{500\frac{cd^2}{m^2}}\right)^{1.6}$$

wherein $L_0$ denotes the initial luminance at the applied current density.

The values correspond to the average of several pixels (typically two to eight), the standard deviation between these pixels is given. Figures show the data series for one OLED pixel.

**Example 1**

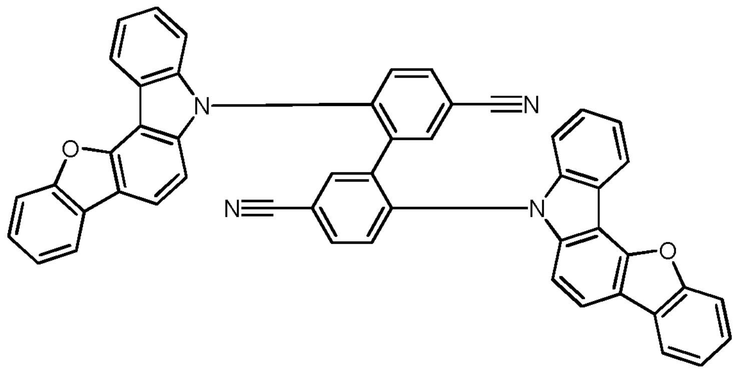

Example **1** was synthesized according to *general synthesis* (53.6 % yield).
Figure 1 depicts the emission spectrum of example **1** (10% by weight in PMMA). The emission maximum is at 447 nm. The photoluminescence quantum yield (PLQY) is 73 % and the full width at half maximum is 0.38 eV and the emission lifetime is 66 µs. The CIEx value is 0.15 and CIEy value is 0.10.
The emission maximum of example **1** in DPEPO (10% by weight) is at 451 nm. The photoluminescence quantum yield (PLQY) is 78 % and the full width at half maximum is 0.38 eV and the emission lifetime is 48 µs. The CIEx value is 0.15 and CIEy value is 0.11.

**Example 2**

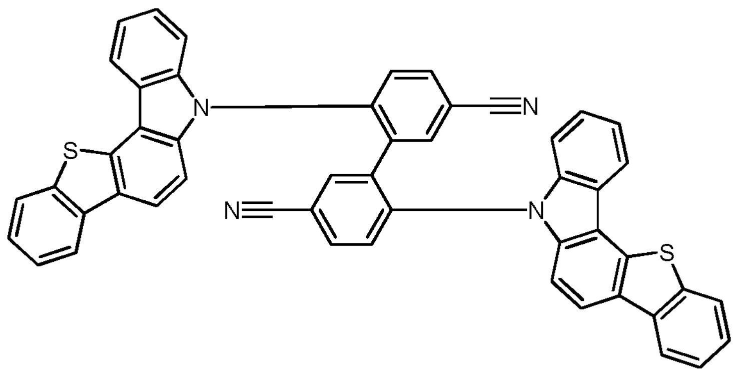

Example **2** was synthesized according to *general synthesis.*
Figure 2 depicts the emission spectrum of example **2** (10% by weight in PMMA). The emission maximum is at 447 nm. The photoluminescence quantum yield (PLQY) is 68 % and the full width at half maximum is 0.38 eV and the emission lifetime is 4 µs. The CIEx value is 0.15 and CIEy value is 0.10.

**Example D1**

Example **1** was tested in the OLED **D1**, which was produced with the following layer structure:

| Layer | Thickness | D1 |
|---|---|---|
| **9** | 100 nm | Al |
| **8** | 2 nm | Liq |
| **7** | 20 nm | NBPhen |
| **6** | 10 nm | MAT1 |
| **5** | 50 nm | Example **1** (20%) : mCBP (80%) |
| **4** | 10 nm | mCBP |

(continued)

| Layer | Thickness | D1 |
|---|---|---|
| 3 | 10 nm | TCTA |
| 2 | 45 nm | NPB |
| 1 | 50 nm | ITO |
| substrate | | glass |

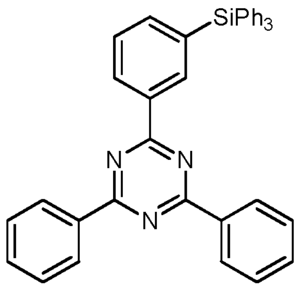

MAT1

Device **D1** yielded an external quantum efficiency (EQE) at 1000 cd/m$^2$ of 11.4%. The emission maximum is at 461 nm with a FWHM of 57 nm at 5 V. The corresponding CIEx value is 0.14 and CIEy is 0.125. The turn-on voltage is 2.7 V. The device shows high lifetime values.

Device **D1** shows an exceptionally good efficiency for the deep-blue color expressed by the CIE values.

**Figures**

Figure 1 Emission spectrum of example **1** (10 % by weight) in PMMA.
Figure 2 Emission spectrum of example **2** (10 % by weight) in PMMA.

**Claims**

**1.** Organic light-emitting molecules comprising or consisting of

- a first chemical moiety comprising or consisting of a structure of Formula I,

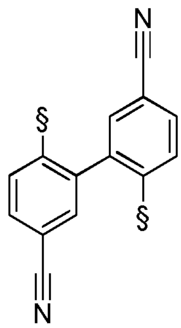

Formula I

and
- two second chemical moieties, each independently from another comprising or consisting of a structure of

Formula II,

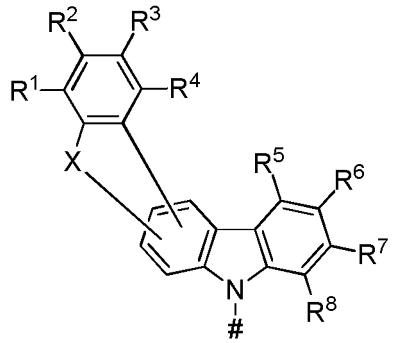

Formula II

wherein the first chemical moiety is linked to each of the two second chemical moieties via a single bond; wherein
§ represents the binding site of a single bond linking the first chemical moiety to one of the two second chemical moieties;
# represents the binding site of a single bond linking one of the two second chemical moieties to the first chemical moiety;
X is O or S;
$R^1$, $R^2$, $R^3$, $R^4$, $R^5$, $R^6$, $R^7$ and $R^8$ is independently from another selected from the group consisting of hydrogen,
deuterium,
$C_1$-$C_5$-alkyl, wherein one or more hydrogen atoms are optionally substituted by deuterium; $C_6$-$C1_8$-aryl, which is optionally substituted with one or more substituents $R^9$; and $C_3$-$C_{17}$-heteroaryl, which is optionally substituted with one or more substituents $R^9$;
$R^9$ is at each occurrence independently from another selected from the group consisting of deuterium, $C_1$-$C_5$-alkyl, wherein one or more hydrogen atoms are optionally substituted by deuterium; and $C_6$-$C_{18}$-aryl, wherein one or more hydrogen atoms are optionally substituted by deuterium.

**2.** The organic light-emitting molecule according to claim 1, wherein the two second chemical moieties, each at each occurrence independently from another comprise or consist of a structure of Formula IIa:

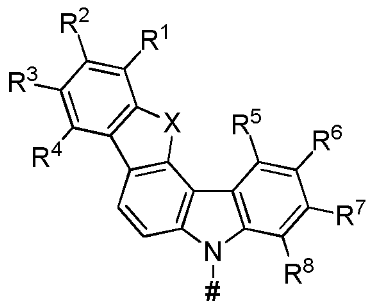

Formula IIa

wherein #, X, $R^1$, $R^2$, $R^3$, $R^4$, $R^5$, $R^6$, $R^7$ and $R^8$ are defined as in claim 1.

**3.** The organic light-emitting molecule according to claim 1 or 2, wherein X is O.

**4.** The organic light-emitting molecule according to one or more of claims 1 to 3, wherein $R^1$, $R^2$, $R^3$, $R^4$, $R^5$, $R^6$, $R^7$ and $R^8$ is independently from another selected from the group consisting of H, Me, $^i$Pr, $^t$Bu and
Ph, which is optionally substituted with one or more substituents independently from each other selected from the group consisting of Me, $^i$Pr, $^t$Bu and Ph;

5. The organic light-emitting molecule according to one or more of claims 1 to 4, wherein $R^1$, $R^2$, $R^3$, $R^4$, $R^5$, $R^6$, $R^7$ and $R^8$ is each H.

6. Use of an organic light-emitting molecule according to one or more of claims 1 to 5 as luminescent emitter and/or host material and or electron transport material and/or hole injection material and/or hole blocking material in an organic optoelectronic device.

7. Use according to claim 6, wherein the organic optoelectronic device is selected from the group consisting of:

• organic light-emitting diodes (OLEDS),
• light-emitting electrochemical cells,
• OLED-sensors, in particular in non-hermetically shielded gas and vapor sensors,
• organic diodes,
• organic solar cells,
• organic transistors,
• organic field-effect transistors,
• organic lasers and
• down-conversion elements.

8. Composition comprising or consisting of:

(a) at least one organic molecule according to one or more of claims 1 to 5, in particular in the form of an emitter and/or a host, and
(b) one or more emitter and/or host materials, which differ from the organic molecule of one or more of claims 1 to 5 and
(c) optional one or more dyes and/or one or more solvents.

9. Organic optoelectronic device, comprising one organic molecule according to one or more of claims 1 to 5 or a composition according to claim 8, in particular in form of a device selected from the group consisting of organic light-emitting diode (OLED), light-emitting electrochemical cell OLED-sensor, in particular in non-hermetically shielded gas and vapor sensors, organic diode, organic solar cell, organic transistor, organic field-effect transistor, organic laser and down-conversion element.

10. Organic optoelectronic device according to claim 9, comprising

- a substrate,
- an anode and
- a cathode, wherein the anode or the cathode are disposed on the substrate and
- at least a light-emitting layer, which is arranged between anode and cathode and which comprises the organic molecule according to claims 1 to 5 or a composition according to claim 8.

11. Process for producing an organic optoelectronic device, wherein an organic molecule according to any one of claims 1 to 5 or a composition according to claim 8 is used, in particular comprising the processing of the organic compound by vacuum evaporation method or from a solution.

## Patentansprüche

1. Organische lichtemittierende Moleküle, aufweisend oder bestehend aus

- einer ersten chemischen Einheit aufweisend eine oder bestehend aus einer Struktur der Formel I

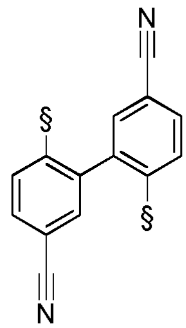

Formel I

und

- zwei zweiten chemischen Einheiten, die jeweils unabhängig voneinander eine Struktur der Formel II aufweisen oder daraus bestehen,

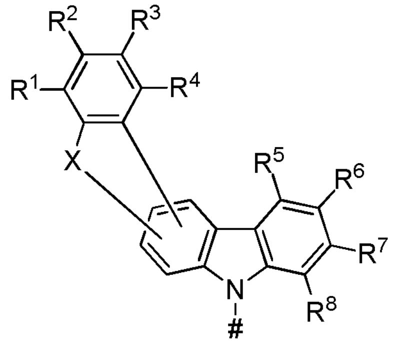

Formel 2

wobei die erste chemische Einheit zu jeder der zwei zweiten chemischen Einheiten über eine Einfachbindung gebunden ist;
wobei
§ steht für die Bindungsstelle einer Einfachbindung, die die erste chemische Einheit mit einer der zwei zweiten chemischen Einheiten verbindet;
# steht für die Bindungsstelle einer Einfachbindung, die einer der zwei zweiten chemischen Einheiten mit der ersten chemischen Einheit verbindet;
X ist O oder S;
$R^1$, $R^2$, $R^3$, $R^4$, $R^5$, $R^6$, $R^7$ und $R^8$ sind unabhängig voneinander ausgewählt aus der Gruppe bestehend aus Wasserstoff,
Deuterium,
$C_1$-$C_5$-Alkyl, wobei eine oder mehrere Wasserstoffatome optional durch Deuterium substituiert sind;
$C_6$-$C_{18}$-Aryl, welches optional mit einem oder mehreren Substituenten $R^9$ substituiert ist; und $C_3$-$C_{17}$-Heteroaryl, welches optional mit einem oder mehreren Substituenten $R^9$ substituiert ist;
$R^9$ ist bei jedem Auftreten unabhängig voneinander ausgewählt aus der Gruppe bestehend aus
Deuterium,
$C_1$-$C_5$-Alkyl, wobei ein oder mehrere Wasserstoffatome optional durch Deuterium substituiert sind; und
$C_6$-$C_{18}$-Aryl, wobei ein oder mehrere Wasserstoffatome optional durch Deuterium substituiert sind.

**2.** Organisches lichtemittierendes Molekül nach Anspruch 1, wobei die zwei zweiten chemischen Einheiten jede bei jedem Auftreten unabhängig voneinander eine Struktur der Formel IIa aufweisen oder daraus bestehen:

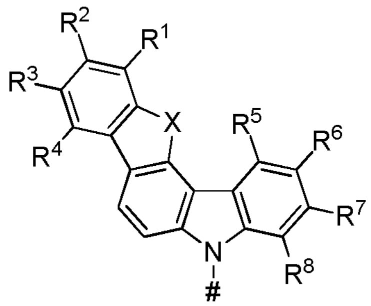

Formel IIa

wobei #, X, $R^1$, $R^2$, $R^3$, $R^4$, $R^5$, $R^6$, $R^7$ und $R^8$ wie in Anspruch 1 definiert sind.

**3.** Organisches lichtemittierendes Molekül nach Anspruch 1 und 2, wobei X gleich O ist.

**4.** Organisches lichtemittierendes Molekül nach einen oder mehreren der Ansprüche 1 bis 3, wobei $R^1$, $R^2$, $R^3$, $R^4$, $R^5$, $R^6$, $R^7$ und $R^8$ unabhängig voneinander ausgewählt sind aus der Gruppe bestehend aus H, Me, $^i$Pr, $^t$Bu und Ph, welches optional substituiert ist mit einem oder mehreren Substituenten, die unabhängig voneinander auswählt sind aus der Gruppe bestehend aus Me, $^i$Pr, $^t$Bu und Ph.

**5.** Organisches lichtemittierendes Molekül nach einen oder mehreren der Ansprüche 1 bis 4, wobei $R^1$, $R^2$, $R^3$, $R^4$, $R^5$, $R^6$, $R^7$ und $R^8$ jeweils H sind.

**6.** Verwendung eines organischen lichtemittierenden Moleküls nach einem oder mehreren der Ansprüche 1 bis 5 als lumineszierender Emitter und/oder Wirtsmaterial und/oder Elektrontransportmaterial und/oder Lochinjektionsmaterial und/oder Lochblockierungsmaterial in einer organischen optoelektronischen Vorrichtung.

**7.** Verwendung nach Anspruch 6, wobei die organische optoelektronische Vorrichtung ausgewählt ist aus der Gruppe bestehend aus

- organischen lichtemittierenden Dioden (OLEDs),
- lichtemittierenden elektrochemischen Zellen,
- OLED-Sensoren, insbesondere in nicht hermetisch abgeschirmten Gas- und Dampfsensoren,
- organischen Dioden,
- organischen Solarzellen,
- organischen Transistoren,
- organischen Feldeffekttransistoren,
- organischen Lasern und
- Down-Konversionselementen.

**8.** Zusammensetzung, aufweisend oder bestehend aus:

(a) mindestens einem organischen Molekül nach einem oder mehreren der Ansprüche 1 bis 5, insbesondere in der Form eines Emitters und/oder Hosts und
(b) einem oder mehreren Emitter- und/oder Wirtsmaterialen, welche sich von den organischen Molekülen nach einem oder mehreren Ansprüche 1 bis 5 unterscheiden und
(c) optional einem oder mehreren Farbstoffen und/oder einem oder mehreren Lösemitteln.

**9.** Organische optoelektronische Vorrichtungen, aufweisend ein organisches Molekül nach einem oder mehreren der Ansprüche 1 bis 5 oder eine Zusammensetzung nach Anspruch 8, insbesondere in Form einer Vorrichtung ausgewählt aus der Gruppe bestehend aus organischer lichtemittierender Diode (OLED), lichtemittierender elektrochemischer Zelle, OLED-Sensor, insbesondere nicht hermetisch abgeschlossen Gas- und Dampfsensoren, organischer Diode, organischer Solarzelle, organischem Transistor, organischem Feldeffekttransistor, organischem Laser und Down-Konversionselement.

**10.** Organische optoelektronische Vorrichtung nach Anspruch 9, aufweisend

- ein Substrat
- eine Anode und
- eine Kathode, wobei die Anode oder die Kathode auf dem Substrat aufgebracht sind und
- mindestens eine lichtemittierende Schicht, welche zwischen der Anode und der Kathode angeordnet ist und welche das organische Molekül nach Anspruch 1 bis 5 oder eine Zusammensetzung nach Anspruch 8 umfasst.

**11.** Verfahren zur Herstellung einer organischen optoelektronischen Vorrichtung, wobei ein organisches Molekül nach einem der Ansprüche 1 bis 5 oder einer Zusammensetzung nach Anspruch 1 verwendet wird, insbesondere umfassend die Prozessierung des organischen Moleküls mittels eines Vakuumverdampfungsverfahrens oder aus einer Lösung.

**Revendications**

**1.** Molécules électroluminescentes organiques comprenant ou constituées de

- un premier fragment chimique comprenant ou constitué d'une structure de formule I,

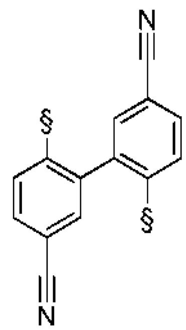

Formule I

et
- deux deuxièmes fragments chimiques, chacun indépendamment des autres, comprenant ou constitués d'une structure de formule II,

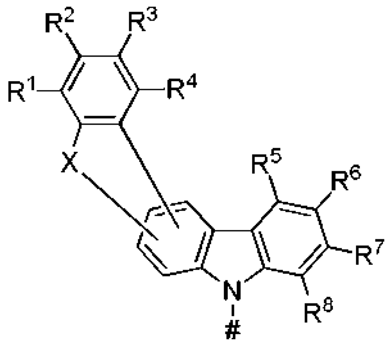

Formule II

dans lesquelles le premier fragment chimique est lié à chacun des deux deuxièmes fragments chimiques par l'intermédiaire d'une simple liaison ;
dans lesquelles
§ représente le site de liaison d'une simple liaison reliant le premier fragment chimique à l'un des deux deuxièmes fragments chimiques ;
# représente le site de liaison d'une simple liaison reliant l'un des deux deuxièmes fragments chimiques au premier fragment chimique ;
X est O ou S ;
$R^1$, $R^2$, $R^3$, $R^4$, $R^5$, $R^6$, $R^7$ et $R^8$ sont, indépendamment les uns des autres, choisis dans le groupe constitué de

hydrogène,
deutérium,
alkyle en $C_1$-$C_5$, dans lequel un ou plusieurs atomes d'hydrogène sont facultativement substitués par deutérium ;
aryle en $C_6$-$C_{18}$, qui est facultativement substitué par un ou plusieurs substituants $R^9$ ; et
hétéroaryle en $C_3$-$C_{17}$, qui est facultativement substitué par un ou plusieurs substituants $R^9$ ;
$R^9$ est, à chaque occurrence, indépendamment des autres, choisi dans le groupe constitué de deutérium,
alkyle en $C_1$-$C_5$, dans lequel un ou plusieurs atomes d'hydrogène sont facultativement substitués par deutérium ; et
aryle en $C_6$-$C_{18}$, dans lequel un ou plusieurs atomes d'hydrogène sont facultativement substitués par deutérium.

2. Molécule électroluminescente organique selon la revendication 1, dans laquelle les deux deuxièmes fragments chimiques, chacun, à chaque occurrence, indépendamment des autres, comprennent ou sont constitués d'une structure de formule IIa :

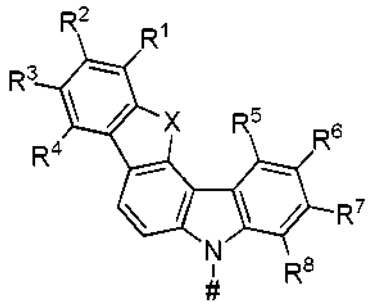

Formule IIa

dans laquelle #, X, $R^1$, $R^2$, $R^3$, $R^4$, $R^5$, $R^6$, $R^7$ et $R^8$ sont tels que définis dans la revendication 1.

3. Molécule électroluminescente organique selon la revendication 1 ou 2, dans laquelle X est O.

4. Molécule électroluminescente organique selon une ou plusieurs des revendications 1 à 3, dans laquelle $R^1$, $R^2$, $R^3$, $R^4$, $R^5$, $R^6$, $R^7$ et $R^8$ sont, indépendamment les uns des autres, choisis dans le groupe constitué de H, Me, $^i$Pr, $^t$Bu et Ph, qui est facultativement substitué par un ou plusieurs substituants, indépendamment les uns des autres, choisis dans le groupe constitué de Me, $^i$Pr, $^t$Bu et Ph.

5. Molécule électroluminescente organique selon un ou plusieurs des revendications 1 à 4, dans laquelle $R^1$, $R^2$, $R^3$, $R^4$, $R^5$, $R^6$, $R^7$ et $R^8$ sont chacun H.

6. Utilisation d'une molécule électroluminescente organique selon un ou plusieurs des revendications 1 à 5 en tant qu'émetteur luminescent et/ou matériau hôte et ou matériau de transport d'électrons et/ou matériau d'injection de trous et/ou matériau de blocage de trous dans un dispositif optoélectronique organique.

7. Utilisation selon la revendication 6, dans laquelle le dispositif optoélectronique organique est choisi dans le groupe constitué de :

- diodes électroluminescentes organiques (OLED),
- cellules électrochimiques électroluminescentes,
- capteurs OLED, en particulier dans des capteurs de gaz et de vapeur non hermétiquement scellés,
- diodes organiques,
- cellules solaires organiques,
- transistors organiques,
- transistors à effet de champ organiques,
- lasers organiques et
- éléments de conversion descendante.

**8.** Composition comprenant ou constituée de :

(a) au moins une molécule organique selon un ou plusieurs des revendications 1 à 5, en particulier sous la forme d'un émetteur et/ou d'un hôte, et
(b) un ou plusieurs matériaux émetteurs et/ou hôtes, qui diffèrent de la molécule organique selon une ou plusieurs des revendications 1 à 5 et
(c) facultativement, un ou plusieurs colorants et/ou un ou plusieurs solvants.

**9.** Dispositif optoélectronique organique, comprenant une molécule organique selon une ou plusieurs des revendications 1 à 5 ou une composition selon la revendication 8, en particulier sous la forme d'un dispositif choisi dans le groupe constitué d'une diode électroluminescente organique (OLED), un capteur OLED à cellule électrochimique électroluminescente, en particulier dans des capteurs de gaz et de vapeur non hermétiquement scellés, une diode organique, une cellule solaire organique, un transistor organique, un transistor à effet de champ organique, un laser organique et un élément de conversion descendante.

**10.** Dispositif optoélectronique organique selon la revendication 9, comprenant

- un substrat,
- une anode et
- une cathode, dans lequel l'anode ou la cathode sont disposées sur le substrat et
- au moins une couche électroluminescente, qui est agencée entre l'anode la cathode et qui comprend la molécule organique selon les revendications 1 à 5 ou une composition selon la revendication 8.

**11.** Procédé de production d'un dispositif optoélectronique organique, dans lequel une molécule organique selon l'une quelconque des revendications 1 à 5 ou une composition selon la revendication 8 est utilisée, en particulier comprenant le traitement du composé organique par un procédé d'évaporation sous vide ou à partir d'une solution.

**Figure 1**

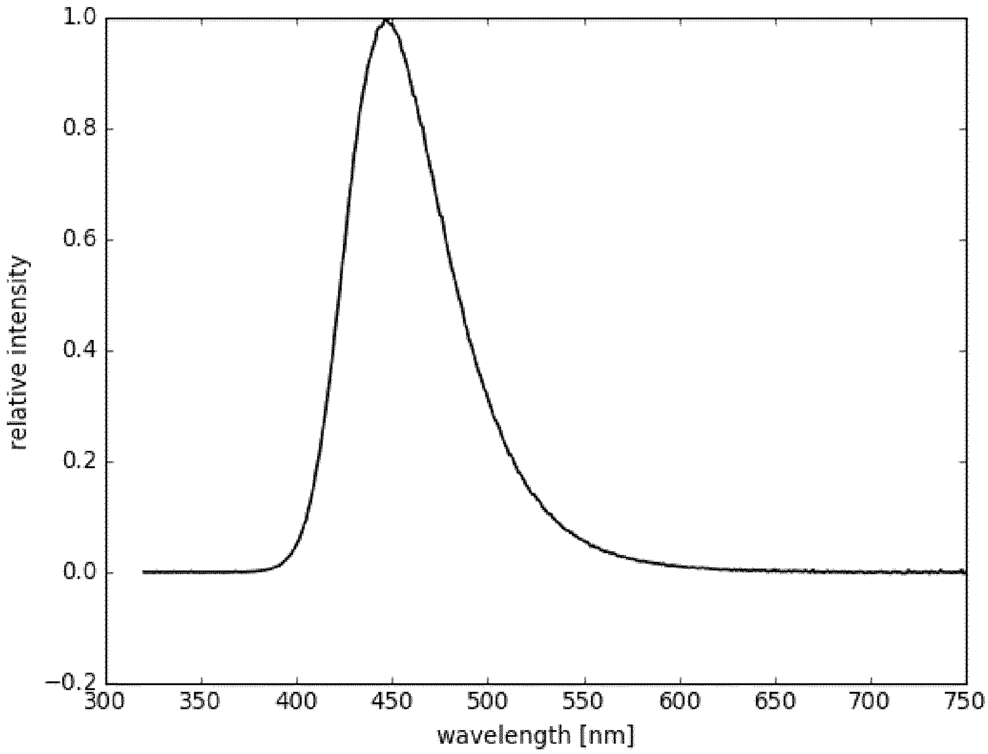


**Figure 2**

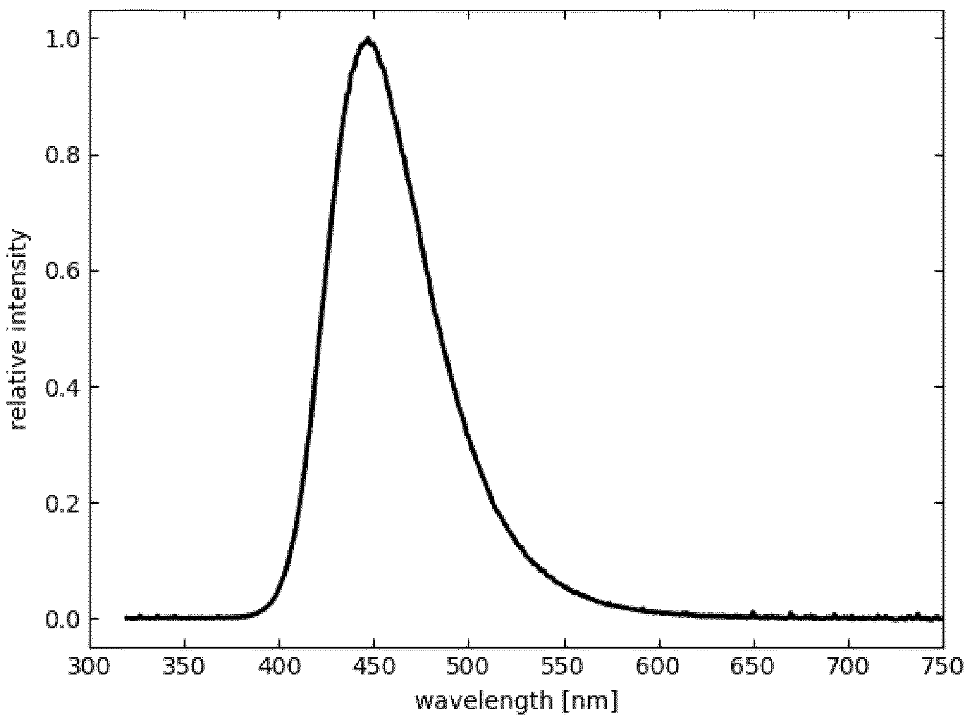

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3113239 A